# FASCICULE DE BREVET EUROPEEN

(11) **EP 0 487 410 B1**
(45) Date de publication et mention de la délivrance du brevet: **11.08.1999**
(21) Numéro de dépôt: 91403118.2
(22) Date de dépôt: 20.11.1991
(51) Int. Cl.: C07K 14/46, A61K 38/17

(54) **Antagonistes d'endothéline**
Endothelin-Antagonisten
Endothelin antagonists

(30) Priorité: 21.11.1990 FR 9014498
(43) Date de publication de la demande: 27.05.1992
(73) Titulaire: HOECHST MARION ROUSSEL, 92800 Puteaux (FR)
(72) Inventeur: Hamon, Gilles, F-93340 Le Raincy (FR); Mahe, Eve, 825, Avenue du Comté de Nice, F-34080 Montpellier (FR); Le-Nguyen, Dung, F-34080 Montpellier (FR)
(74) Mandataire: Vieillefosse, Jean-Claude

(56) Documents cités:
- EP-A- 0 402 313
- WO-A-92/02237
- BIOCHEMICAL AND BIOPHYSICAL RESEARCH COMMUNICATIONS, vol. 163, no. 1, 30 août 1989, pages 424-429, Academic Press, Inc.; K. NAKAJIMA et al.: "Structure-activity relationship of endothelin: Importance of charged groups"
- FEBS, vol. 256, nos 1,2, octobre 1989, pages 1-3, Elsevier Science Publishers B.V. (Biomedical Division); A. BDOLAH et al.: "SRTX-d, a new native peptide of the endothelin/sarafotoxin family"
- BIOCHEMICAL AND BIOPHYSICAL RESEARCH COMMUNICATIONS, vol. 160, no. 1, 1989, pages 228-234, Academic Press, Inc.; Y. HIRATA et al.: "Receptor binding activity and cytosolic free calcium response by synthetic endothelin analogs in cultured rat vascular smooth muscle cells"
- JOURNAL OF CARDIOVASCULAR PHARMACOLOGY, vol. 13 (suppl. 5), 9th March 1989, pages 5197-5199, Raven Press Ltd, New York, US; C.R. HILEY et al.: "Pressor effects of endothelin-1 and some analogs in the perfused superior mesenteric arterial bed of the rat"
- JOURNAL OF THE AMERICAN CHEMICAL SOCIETY, vol. 105, 1983, pages 6442-6455, American Chemical Society; J.P. TAM et al.: "SN2 deprotection of synthetic peptides with a low concentration of HF in dimethyl sulfide: evidence and application in peptide synthesis"

## Description

La présente demande concerne de nouveaux dérivés peptidiques, le procédé de préparation, l'application à titre de médicament de ces nouveaux dérivés et les compositions pharmaceutiques les renfermant.

On sait que l'endothéline est un puissant vasoconstricteur extrait à partir d'endothélium aortique de porc. Ce peptide qui a été isolé récemment, comporte 21 amino acides, présente 2 ponts disulfures et a pour formule : 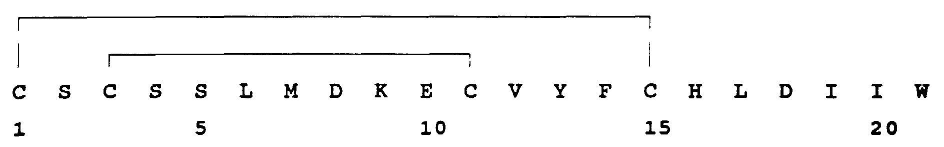

C S C S S L M D K E C V Y F C H L D I I W

1 5 10 15 20
 (- A novel potent vasoconstrictor peptide produced by vascular endothelial cells. M. Yanagisawa, H. Kurihara, S. Kimura, Y. Tomobe, M. Kobayashi, Y. Mitsui, Y. Yazaki, K. Goto & T. Masaki, Nature, (1988) 332, p. 411-415). Ce peptide est désigné dans ce qui suit sous le nom de ET1.

La nomenclature utilisée est celle de la commission IUPAC-IUB (1984) European J. Biochem 183, 9-37.

Ce peptide qui renferme 21 résidus d'amino-acides, 2 ponts disulfures est capable de contracter à de très faibles doses les cellules des muscles lisses (artères et veines) de différentes espèces de mammifères (homme, chien, chat, cochon, cobaye, rat, lapin..). En essayant de raccourcir la chaine et de remplacer certains restes d'amino-acides de l'endothéline par d'autres amino-acides, la demanderesse a préparé par synthèse, de nouveaux dérivés peptidiques dans lesquels l'activité vasoconstrictrice de l'endothéline a pratiquement disparu et qui présente de plus, la propriété de bloquer et de se fixer sur les récepteurs naturels de l'endothéline. Les produits présentent ainsi une activité qui s'opposera à toute action d'une libération ultérieure de l'endothéline.

Ces produits présentent en outre, l'intérêt de pouvoir être préparés par synthèse totale en grandes quantités.

L'article "Biochemical and Biophysical Research Communications", vol. 163, n°1, 30 août 1989, Nakajima et al, "Structure-activity relationship of endothelin : Importance of charged groups" décrit l'importance des groupes COOH et NH₂ pour la formation de la liaison entre ET et son récepteur.

L'article FEBS, vol. 256, n°1-2, Octobre 1989, Bdolah et al, "SRTX-d, a new native peptide of the endothelin/ sarafotoxin family" décrit l'importance de la substitution en position 2 (Ser/Thr).

EP-A-0402313 document intercalaire, décrit la [NLe-7]-ET1; il n'y a pas de mention de la suppression de l'activité vasoconstrictrice.

WO-A-9202237 décrit certains dérivés de l'endothéline.

La présente demande a ainsi pour objet de nouveaux dérivés peptidiques répondant à la formule générale (I) :

R1 - H X₁ D X₂ I X₃ (I)

dans laquelle X₁ représente un résidu de leucine, d'arginine ou de glutamine, X₂ représente un résidu d'isoleucine ou de valine, X₃ représente un résidu de tryptophane, d'amidotryptophane ou de D-naphtylalanine et R1 représente un reste de 15 amino-acides de formule (Ia) : 
C S C S S L X4 D K E C X5 Y F C -          (Ia)
1       5         10
 dans lequel X₄ en position 7 représente un résidu de glycine, de norleucine, de valine, d'isoleucine, de leucine, d'alanine, de phénylalanine, de béta-alanine ou de méthionine, X₅ en position 12 représente un résidu de valine, de leucine ou d'acide glutamique et le trait pointillé représente le cas échéant un pont disulfure entre 2 résidus de cystéine, étant entendu que X₄ ne peut représenter un résidu de méthionine lorsque X₅ représente un résidu de valine,
à l'exclusion du dérivé [Nle-7]-ET1.

Des dérivés peptidiques font l'objet des revendications dépendantes 2 à 4.

L'invention a également pour objet, un procédé de préparation des nouveaux dérivés peptidiques tels que définis ci-dessus, caractérisé en ce que l'on effectue une synthèse en phase solide en introduisant séquentiellement sur un support de type polystyrène réticulé les amino-acides dûment protégés, à l'aide d'un agent de couplage, déprotège les amino-acides, et libère de la résine la chaîne peptidique ainsi formée, puis cyclise le cas échéant, le ou les ponts disulfures pour obtenir le dérivé peptidique ainsi recherché.

Dans des conditions préférentielles de mise en oeuvre, le procédé ci-dessus décrit est caractérisé en ce que :
- le support de type polystyrène réticulé est une résine de type "Boc-Trp-CM" dans laquelle Boc est un groupement tertiobutyloxycarbonyl, Trp est un groupement tryptophyl et CM désigne le support de polystyrène réticulé,
- l'agent de couplage est le (benzotriazol-1-yloxy) tris(diméthylamino) phosphonium hexafluorophosphate ou Bop,
- la libération du support de la chaîne peptidique ainsi que la déprotection des amino-acides est effectuée au moyen de l'acide fluorhydrique en opérant à basse température de préférence vers 0°C.

Les nouveaux dérivés peptidiques, objet de la présente invention présentent de très intéressantes propriétés pharmacologiques ; on note en particulier un remarquable effet inhibiteur contre l'hypertension induite par l'endothéline, un effet antiischémique. L'activité vasoconstrictrice de l'endothéline a par contre disparu.

Certaines de ces propriétés sont illustrées plus loin dans la partie expérimentale.

Ces propriétés justifient l'utilisation des nouveaux dérivés peptidiques répondant à la formule (I) ci-dessus à titre de médicaments.

La présente invention a ainsi également pour objet, l'application à titre de médicaments des nouveaux dérivés peptidiques tels que définis par la formule générale (I) ci-dessus et objet des revendications de produit.

Ces médicaments trouvent, par exemple, leur emploi dans le traitement de tous les spasmes vasculaires, dans le traitement des post-hémorragies cérébrales, dans le traitement des spasmes coronariens, des spasmes vasculaires périphériques ainsi que dans le traitement des insuffisances rénales.

La dose usuelle, variable selon le produit utilisé, le sujet traité et l'affection en cause, peut être par exemple de 1 à 300 mg par jour par voie intraveineuse chez l'homme.

L'invention a également pour objet les compositions pharmaceutiques qui renferment au moins un dérivé précité à titre de principe actif.

A titre de principe actif, les dérivés peptidiques répondant à la formule générale (I), peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables, les aérosols ; elles sont préparées selon les méthodes usuelles. Le ou les principes actifs peuvent être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Il va être donné maintenant à titre non limitatif, des exemples de mise en oeuvre de l'invention.

### EXEMPLE 1 : Dérivé [ Nle-7] -ET1

### STADE A : Assemblage de la chaîne peptidique

On utilise un support de type "Boc-Trp-CM-support" dans laquelle Boc est un groupement tertiobutyloxycarbonyl, Trp représente un groupement tryptophyl et CM-support désigne le support de polystyrène réticulé.

Le support renfermant 0,6 mmol Trp/g a été préparé par la méthode au fluorure de potassium selon la technique décrite par Horiki K., Igano K. & Inouye K.(1978) Chemistry Lett., 165-168.

Tous les aminoacides sont N-protégés par le groupement tertiobutylcarbonyl, les groupements protecteurs des chaînes latérales ont été les suivants :
- p-méthyl benzyl pour la cystéine,
- ester de cyclohexyl pour l'acide aspartique et l'acide glutamique,
- benzyl pour la sérine,
- o-chlorobenzyloxycarbonyl pour la lysine,
- 2,6-dichlorobenzyl pour la tyrosine,
- Nim-tertiobutyloxycarbonyl,
- le tryptophane et la méthionine ne sont pas protégés.

Les réactions de couplage cnt été effectuées en utilisant le Bop ou (benzotriazol-1-yloxy tris (diméthylamino) phosphonium hexafluorophosphate.

Un cycle de couplage peut être résumé comme suit :
Déprotection :
1 - Traitement avec une solution d'acide trifluoroacétique (50 %) dans le dichlorométhane contenant 3 % d'éthanedithiol pendant 1 minute.
2 - Vidange puis traitement avec une solution d'acide trifluoroacétique à 50 % dans le dichlorométhane contenant 3 % d'éthanedithiol pendant 30 minutes.
3 - Vidange puis lavage avec de l'isopropanol contenant 5 % d'éthanedithiol.
4 - Lavage au dichlorométhane, 2 fois.
Couplage :
1 - Addition de Bop et de Boc-acide aminé.
2 - Addition de diisopropyléthylamine (6 équivalents) puis de solvant (dichlorométhane ou diméthylformamide) sous agitation.
3 - Après réaction négative à la ninhydrine lavage 2 fois au dichlorométhane.

Les tests de contrôle utilisant la ninhydrine ont été effectués suivant le procédé décrit par Kaiser, E., Colescott, R.L., Bossinger, C.D. & Cook, P.I. (1970) Anal. Biochem. 24, 595-598.

Avant le traitement par l'acide fluorhydrique le dernier groupement Boc est éliminé par l'acide trifluoroacétique. Au départ de 2 g de résine " Boc-Trp-CM-résine" on a ainsi obtenu en opérant manuellement 5,25 g de complexe chaîne peptidique protégée, résine que l'on utilise directement au stade suivant.

### STADE B : Libération du complexe chaîne peptidique protégée-résine

On fait réagir 1,5 g de complexe chaîne peptidique protégée-résine pendant 60 minutes avec 15 ml d'acide fluorhydrique à 0°C en présence d'anisole (1 ml) et diméthylsulfide (0,5 ml). La résine est lavée à l'éther ; le peptide brut est extrait par une solution aqueuse d'acide acétique à 20 %. Après lyophilisation, on obtient 660 mg de produit brut non cyclisé que l'on utilise directement au stade suivant.

### STADE C : Cyclisation et purification

On dissout 500 mg du peptide brut obtenu ci-dessus, dans 250 ml d'eau, agite vigoureusement, ajoute de la diisopropyl éthylamine jusqu'à obtention de pH 8 (une goutte de mélange est recueillie toutes les heures et ajoutée à une goutte d'une solution contenant de l'acide dithiobis (2-nitrobenzoique) dans un tampon molaire de K₂HPO₄ (pH 8) pour suivre la réaction d'oxydation). Pendant toute la réaction on maintient le pH à 8 par addition de diisopropyléthylamine. La réaction est suivie par chromatographie liquide haute performance (HPLC).

Après 28 heures, on constate l'absence de coloration jaune dans le test à l'acide dithiobis (2-nitrobenzoique).

Le produit obtenu est purifié par chromatographie liquide haute performance (HPLC) sur colonne Whatman M20 ODS₃ et élué par un mélange eau-acétonitrile renfermant 0,1 % d'acide trifluoroacétique.

On lyophilise la fraction obtenue et recueille finalement 30 mg de produit attendu.

La composition en aminoacides figure dans le tableau I ci-après :

### EXEMPLES 2 à 15 :

En opérant de la même manière qu'à l'exemple 1 mais avec d'autres amino-acides, on a préparé les 14 autres micro-protéines qui figurent ci-après ; toutefois, pour les produits des exemples 2 à 15, au stade C de cyclisation et de purification, on a utilisé 500 ml d'eau pour 500 mg de peptide.

### EXEMPLE 2 : Dérivé ( Gly-7)-ET1 :

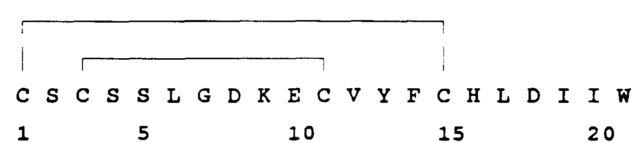
C S C S S L G D K E C V Y F C H L D I I W
1       5         10        15        20

### EXEMPLE 3 : Dérivé (Nle-7)-D-Naphtylalanine-21-ET1

### EXEMPLE 4 : Dérivé (Nle-7)-Amidotryptophane-21-ET1

### EXEMPLE 5 : Dérivé (Beta-alanine-7)-ET1

### EXEMPLE 6 : Dérivé (Val-7)-ET1

### EXEMPLE 12 : Dérivé de formule la (X₄ en 7 = Nle, X₁ = Gln, X₂ = Ile, X₃ = Trp, X₅ = Val Avec deux ponts disulfure entre les cystéines 1 - 15 et 3 - 11)

### EXEMPLE 13 : Dérivé de formule la (X₄ en 7 = Met, X₅ en 12 = Leu, X₁ = Gln, X₂ = Ile, X₃ = Trp Avec deux ponts disulfure entre les cystéines 1 - 15 et 3 - 11)

Les compositions en amino-acides sont indiquées dans le tableau I ci-après.

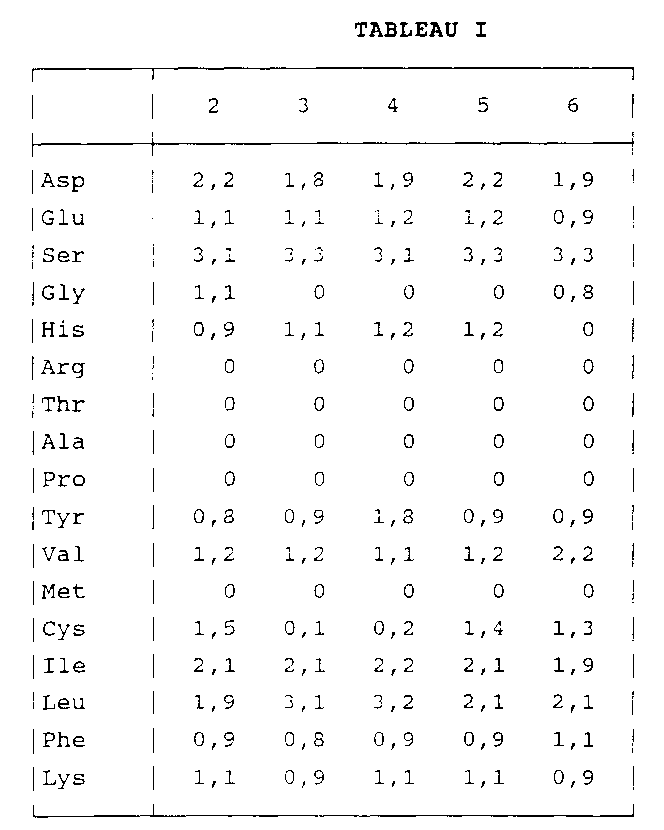

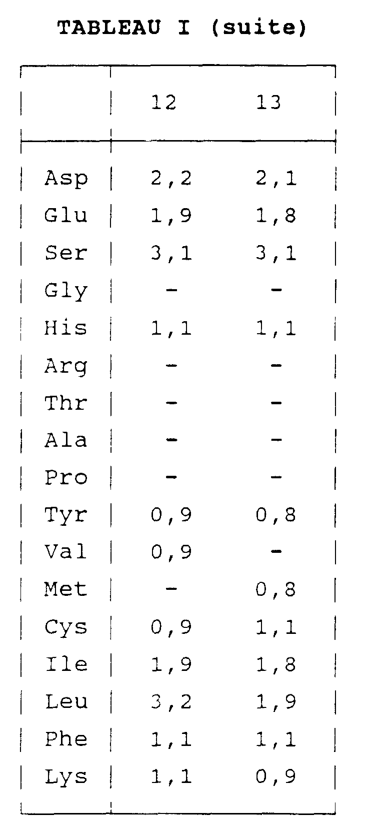

### EXEMPLE 16 :

On a préparé un soluté injectable répondant à la formulation suivante :
- Produit de l'exemple 1 1 mg
- excipient aqueux stérile 2 ml

### EXEMPLE 17 :

On a préparé des comprimés répondant à la formule suivante :
- Produit de l'exemple 1 2 mg
- excipient q.s.p. un comprimé terminé à 150 mg (composition de l'excipient : lactose, amidon, talc, stéarate de magnésium).

### ETUDE DE L'ACTIVITE SUR RECEPTEUR DE L'ENDOTHELINE

On effectue une préparation membranaire à partir de cortex postérieur plus cervelet de rat. Le tissu est broyé au POLYTRON dans un tampon Tris 50 mM pH = 7,4.

Après 30 minutes à 25 °C (B.M.) l'homogénat est centrifugé à 30000 g pendant 15 minutes (2 centrifugations avec reprise intermediaire dans le tampon Tris pH. 7,4).

Les culots sont remis en suspension dans un tampon d'incubation (Tris 25 mM, pepstatine A 5 microg/ml, aprotinine 3 microg/ml, PMSF 0,1 mM, EDTA 3mM, EGTA 1mM pH 7,4).

On répartit des aliquotes de 2 ml dans des tubes à hémolyse et ajoute de la 125I Endothéline (environ 50000 dpm/tube) et le produit à étudier. (Le produit est d'abord testé à 3*10⁻⁵ M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.

La liaison non spécifique est déterminée par addition d'endothéline à 10⁻⁶ M (en triple). On incube à 25 °C pendant 60 minutes, remet au bain-marie à 0 °C, pendant 5 minutes, filtre sous pression réduite, rince au tampon Tris 7,4 et compte la radioactivité en présence du scintillant Triton.

Le résultat est exprimé directement en concentration inhibitrice 50 % (CI50), c'est-à-dire en concentration de produit étudié exprimée en nM, nécessaire pour déplacer 50 % de la radioactivité spécifique fixée sur le récepteur étudié.

### Résultat :

Les CI50 trouves pour les produits des exemples 1 à 15 sont données dans le tableau II ci-après, en nanomoles.

**TABLEAU Il**

| PRODUITS DES EXEMPLES | CI50 (nM) |
|---|---|
| 1 | 10 |
| 2 | 1.4 |
| 3 | 2 |
| 4 | 10 |
| 5 | 2.5 |
| 6 | 5.2 |
| 12 | 2.3 |
| 13 | 3.5 |

## Revendications (Revendications pour l'(les) Etat(s) contractant(s) suivant(s): AT, BE, CH, LI, DE, DK, FR, GB, IT, MC, NL, SE)

1. Dérivés peptidiques répondant à la formule générale (I):
R1 - H X1 D X2 I X3 (I)
dans laquelle X1 représente un résidu de leucine, d'arginine ou de glutamine, X2 représente un résidu d'isoleucine ou de valine, X3 représente un résidu de tryptophane, d'amidotryptophane ou de D-naphtylalanine et R1 représente un reste de 15 amino-acides de formule (Ia) : 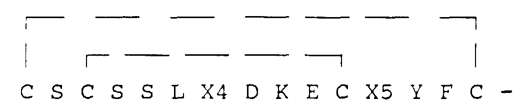
C S C S S L X4 D K E C X5 Y F C -
 dans lequel X4 en position 7 représente un résidu de glycine, de norleucine, de valine, d'isoleucine, de leucine, d'alanine, de phénylalanine, de béta-alanine ou de méthionine, X5 en position 12 représente un résidu de valine, de leucine ou d'acide glutamique et le trait pointillé représente le cas échéant un pont disulfure entre 2 résidus de cystéine, étant entendu que X4 ne peut représenter un résidu de méthionine lorsque X5 représente un résidu de valine;
à l'exclusion du dérivé [Nle-7]-ET1.

2. Dérivés peptidiques selon la revendication 1, caractérisés en ce qu'ils sont constitués par l'un des dérivés peptidiques suivants : 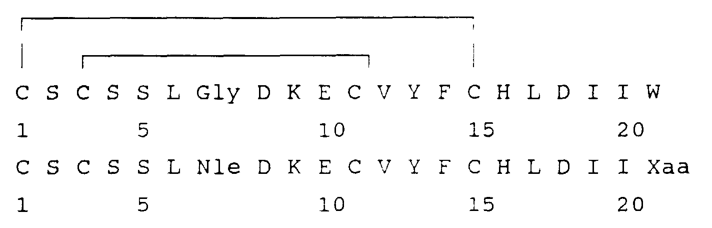
C S C S S L Gly D K E C V Y F C H L D I I W
1       5           10        15        20
C S C S S L Nle D K E C V Y F C H L D I I Xaa
1       5           10        15        20
 où Xaa représente D-Naphtylalanine

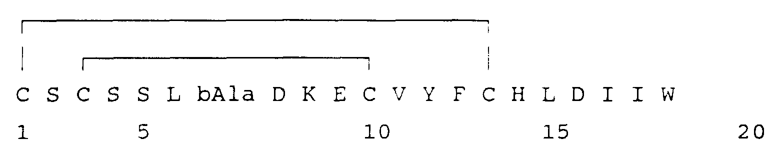
C S C S S L bAla D K E C V Y F C H L D I I W
1       5                 10          15          20

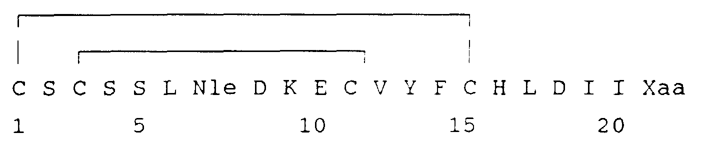
C S C S S L Nle D K E C V Y F C H L D I I Xaa
1 5 10 15 20

C S C S S L M D K E C L Y F C H Q D I I W
1 5 10 15 20

3. Procédé de préparation des nouveaux dérivés peptidiques tels que définis à l'une des revendications 1 ou 2, caractérisé en ce que l'on effectue une synthèse en phase solide en introduisant séquentiellement sur un support de type polystyrène réticulé les amino-acides dûment protégés, à l'aide d'un agent de couplage, déprotège les amino-acides, et libère de la résine la chaîne peptidique ainsi formée, puis cyclise le cas échéant, le ou les ponts disulfures pour obtenir le dérivé peptidique ainsi recherché.

4. Procédé selon la revendication 3, caractérisé en ce que:
- le support de type polystyrène réticulé est une résine de type "Boc-Trp-CM" dans laquelle Boc est un groupement tertio-butyloxycarbonyl, Trp est un groupement tryptophyl et CM désigne le support de polystyrène réticulé,
- l'agent de couplage est le (benzotriazol-l-yloxy) tris-(diméthylamino) phosphonium hexafluorophosphate ou Bop,
- la libération du support de la chaîne peptidique ainsi que la déprotection des amino-acides est effectuée au moyen de l'acide fluorhydrique en opérant à basse température de préférence vers 0°C.

5. Médicaments, caractérisés en ce qu'ils sont constitués par les dérivés peptidiques tels que définis par la formule générale (I) de la revendication 1.

6. Médicaments, caractérisés en ce qu'ils sont constitués par les dérivés peptidiques tels que définis dans la revendication 4.

7. Compositions pharmaceutiques, caractérisées en ce qu'elles renferment à titre de principe actif, l'un au moins des médicaments tels que définis à l'une quelconque des revendications 5 et 6.

8. Utilisation du dérivé [Nle-7]-ET1 pour la préparation d'un médicament pour l'inhibition contre l'hypertension induite par l'endothéline sans effet vasoconstricteur de l'endothéline.

## Revendications (Revendications pour l'(les) Etat(s) contractant(s) suivant(s): ES, GR)

1. Procédé de préparation de dérivés peptidiques répondant à la formule générale (I):
R1 - H X1 D X2 I X3 (I)
dans laquelle X1 représente un résidu de leucine, d'arginine ou de glutamine, X2 représente un résidu d'isoleucine ou de valine, X3 représente un résidu de tryptophane, d'amidotryptophane ou de D-naphtylalanine et R1 représente un reste de 15 amino-acides de formule (Ia) : C S C S S L X4 D K E C X5 Y F C - dans lequel X4 en position 7 représente un résidu de glycine, de norleucine, de valine, d'isoleucine, de leucine, d'alanine, de phénylalanine, de béta-alanine ou de méthionine, X5 en position 12 représente un résidu de valine, de leucine ou d'acide glutamique et le trait pointillé représente le cas échéant un pont disulfure entre 2 résidus de cystéine, étant entendu que X4 ne peut représenter un résidu de méthionine lorsque X5 représente un résidu de valine;
à l'exclusion du dérivé [Nle-7]-ET1, caractérisé en ce que l'on effectue une synthèse en phase solide en introduisant séquentiellement sur un support de type polystyrène réticulé les amino-acides dûment protégés, à l'aide d'un agent de couplage, déprotège les amino-acides, et libère de la résine la chaîne peptidique ainsi formée, puis cyclise le cas échéant, le ou les ponts disulfures pour obtenir le dérivé peptidique ainsi recherché.

2. Procédé selon la revendication 1, caractériséen ce que :
- le support de type polystyrène réticulé est une résine de type "Boc-Trp-CM" dans laquelle Boc est un groupement tertio-butyloxycarbonyl, Trp est un groupement tryptophyl et CM désigne le support de polystyrène réticulé,
- l'agent de couplage est le (benzotriazol-1-yloxy) tris(diméthylamino) phosphonium hexafluorophosphate ou Bop,
- la libération du support de la chaîne peptidique ainsi que la déprotection des amino-acides est effectuée au moyen de l'acide fluorhydrique en opérant à basse température de préférence vers 0°C.

3. Procédé selon la revendication 1, caractérisés en ce que les dérivés peptidiques sont constitués par l'un des dérivés peptidiques suivants : 
C S C S S L Gly D K E C V Y F C H L D I I W
1 5 10 15 20
C S C S S L Nle D K E C V Y F C H L D I I Xaa
1 5 10 15 20
 où Xaa représente D-Naphtylalanine

C S C S S L bAla D K E C V Y F C H L D I I W
1 5 10 15 20

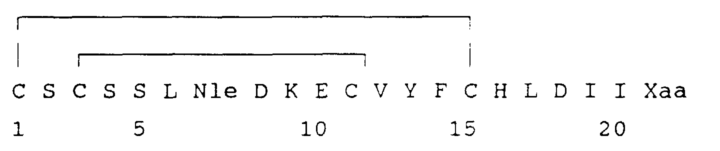
C S C S S L Nle D K E C V Y F C H L D I I Xaa
1       5         10        15        20

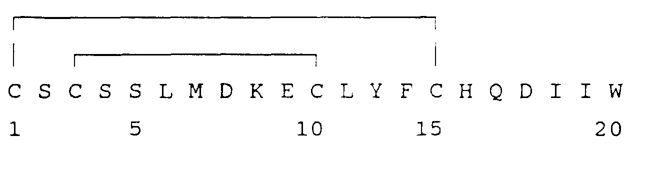
C S C S S L M D K E C L Y F C H Q D I I W
1       5                 10                  15                  20

## Claims (Claims for the following Contracting State(s): AT, BE, CH, LI, DE, DK, FR, GB, IT, MC, NL, SE)

1. New peptide derivatives corresponding to general formula (I):
R1 - H X1 D X2 I X3 (I)
in which X1 represents a residue of leucine, arginine or glutamine, X2 represents a residue of isoleucine or valine, X3 represents a residue of tryptophan, amidotryptophan or D-naphthylalanine and R1 represents a remainder of 15 amino-acids of formula (Ia): C S C S S L X4 D K E C X5 Y F C - in which X4 in position 7 represents a residue of glycine, norleucine, valine, isoleucine, leucine, alanine, phenylalanine, beta-alanine or methionine, X5 in position 12 represents a residue of valine, leucine or glutamic acid and the dotted line represents if appropriate a disulphide bridge between 2 cysteine residues, it being understood that X4 cannot represent a methionine residue when X5 represents a valine residue, with the exception of the [Nle-7]-ET1 derivative.

2. Peptide derivatives according to claim 1, characterized in that they are constituted by one of the following peptide derivatives: 
C S C S S L Gly D K E C V Y F C H L D I I W
1 5 10 15 20
C S C S S L Nle D K E C V Y F C H L D I I Xaa
1 5 10 15 20
 where Xaa represents D-Naphthylalanine

C S C S S L bAla D K E C V Y F C H L D I I W
1 5 10 15 20

C S C S S L Nle D K E C V Y F C H L D I I Xaa
1 5 10 15 20

C S C S S L M D K E C L Y F C H Q D I I W
1       5         10        15        20

3. Preparation process for new peptide derivatives as defined in claim 1 or 2, characterized in that a solid phase synthesis is carried out by introducing sequentially on a support of cross-linked polystyrene type duly protected amino-acids, using a coupling agent, the amino-acids are deprotected, and the peptide chain thus formed is released from the resin, then if appropriate the disulphide bridge or bridges are cyclized in order to obtain the peptide derivative thus sought.

4. Process according to claim 3, characterized in that:
- the support of cross-linked polystyrene type is a "Boc-Trp-CM" type resin in which Boc is a tertio-butyloxycarbonyl group, Trp is a tryptophyl group and CM designates the cross-linked polystyrene support,
- the coupling agent is (benzotriazol-1-yloxy) tris-(dimethylamino)-phosphonium hexafluorophosphate or Bop,
- the release of the support of the peptide chain as well as the deprotection of the amino-acids is carried out using hydrofluoric acid by operating at a low temperature, preferably about 0°C.

5. Medicaments, characterized in that they are constituted by the peptide derivatives as defined by general formula (I) of claim 1.

6. Medicaments, characterized in that they are constituted by the peptide derivatives as defined in claim 4.

7. Pharmaceutical compositions, characterized in that they contain as active ingredient, at least one of the medicaments as defined in one any of claims 5 and 6.

8. Use of the [Nle-7]-ET1 derivative for the preparation of a medicament for the inhibition of hypertension induced by endothelin without the vasoconstrictor effect of endothelin.

## Claims (Claims for the following Contracting State(s): ES, GR)

1. Preparation process for peptide derivatives corresponding to general formula (I):
R1 - H X1 D X2 I X3 (I)
in which X1 represents a residue of leucine, arginine or glutamine, X2 represents a residue of isoleucine or valine, X3 represents a residue of tryptophan, amidotryptophan or D-naphthylalanine and R1 represents a remainder of 15 amino-acids of formula (Ia): C S C S S L X4 D K E C X5 Y F C - in which X4 in position 7 represents a residue of glycine, norleucine, valine, isoleucine, leucine, alanine, phenylalanine, beta-alanine or methionine, X5 in position 12 represents a residue of valine, leucine or glutamic acid and the dotted line represents if appropriate a disulphide bridge between 2 cysteine residues, it being understood that X₄ cannot represent a methionine residue when X5 represents a valine residue;
with the exception of the [Nle-7]-ET1 derivative, characterized in that a solid phase synthesis is carried out by introducing sequentially on a support of cross-linked polystyrene type duly protected amino-acids, using a coupling agent, the amino-acids are deprotected, and the peptide chain thus formed is released from the resin, then if appropriate the disulphide bridge or bridges are cyclized in order to obtain the peptide derivative thus sought.

2. Process according to claim 1, characterized in that:
- the support of cross-linked polystyrene type is a "Boc-Trp-CM" type resin in which Boc is a tertio-butyloxycarbonyl group, Trp is a tryptophyl group and CM designates the cross-linked polystyrene support,
- the coupling agent is (benzotriazol-1-yloxy) tris-(dimethylamino)-phosphonium hexafluorophosphate or Bop,
- the release of the support of the peptide chain as well as the deprotection of the amino-acids is carried out using hydrofluoric acid by operating at a low temperature, preferably about 0°C.

3. Process according to claim 1, characterized in that the peptide derivatives are constituted by one of the following peptide derivatives: 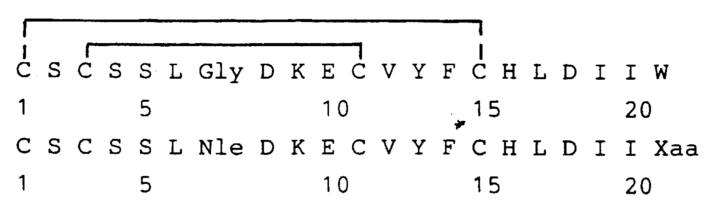
C S C S S L Gly D K E C V Y F C H L D I I W
1       5         10        15        20
C S C S S L Nle D K E C V Y F C H L D I I Xaa
1       5         10        15        20
 where Xaa represents D-Naphthylalanine

C S C S S L bAla D K E C V Y F C H L D I I W
1 5 10 15 20

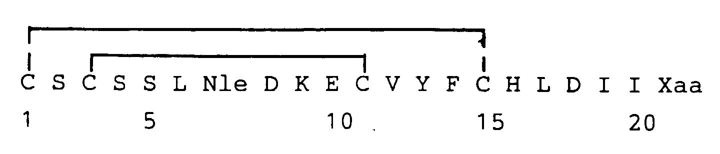
C S C S S L Nle D K E C V Y F C H L D I I Xaa
1 5 10 15 20

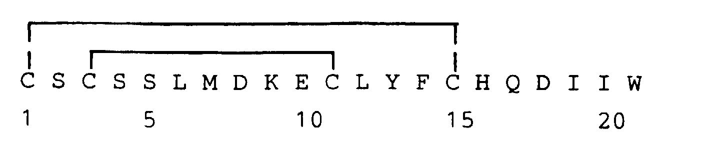
C S C S S L M D K E C L Y F C H Q D I I W
1       5         10        15        20

## Patentansprüche (Patentansprüche für folgende(n) Vertragsstaat(en): AT, BE, CH, LI, DE, DK, FR, GB, IT, MC, NL, SE)

1. Peptidderivate, die der allgemeinen Formel (I):
R1 - H X1 D X2 I X3 (I)
entsprechen, in der X1 einen Leucin-, Arginin- oder Glutaminrest darstellt, X2 einen Isoleucin- oder Valinrest darstellt, X3 einen Tryptophan-, Amidotryptophan- oder D-Naphthylalaninrest darstellt und R1 einen Rest aus 15 Aminosäuren der Formel (la): C S C S S L X4 D K E C X5 Y F C - darstellt, in dem X4 in Position 7 einen Glycin-, Norleucin-, Valin-, Isoleucin-, Leucin-, Alanin-, Phenylalanin-, beta-Alanin- oder Methioninrest darstellt, X5 in Position 12 einen Valin-, Leucin- oder Glutaminsäurerest darstellt und die punktierte Linie gegebenenfalls eine Disulfidbrücke zwischen 2 Cysteinresten darstellt, wobei es sich versteht, daß X4 keinen Methioninrest darstellen kann, wenn X5 einen Valinrest darstellt;
mit Ausnahme des Derivats [Nle-7]-ET1.

2. Peptidderivate gemäß Anspruch 1, dadurch gekennzeichnet, daß sie aus einem der folgenden Peptidderivate bestehen: 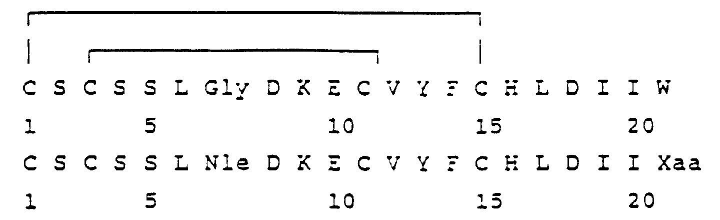
C S C S S L Gly D K E C V Y F C H L D I I W
1       5           10          15          20
C S C S S L Nle D K E C V Y F C H L D I I Xaa
1       5           10          15          20
 worin Xaa D-Naphthylalanin darstellt

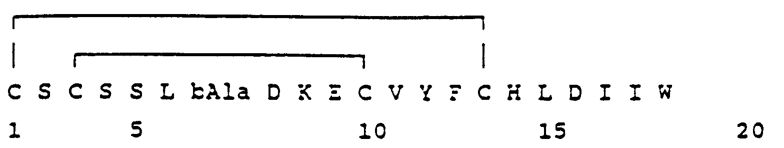
C S C S S L bAla D K E C V Y F C H L D I I W
1       5           10          15          20

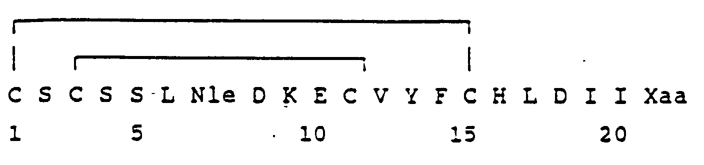
C S C S S L Nle D K E C V Y F C H L D I I Xaa
1       5         10        15        20

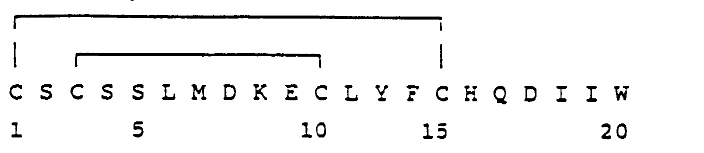

C S C S S L M D K E C L Y F C H Q D I I W

1       5         10    15          20

3. Verfahren zur Herstellung der neuen Peptidderivate wie in einem der Ansprüche 1 oder 2 definiert, dadurch gekennzeichnet, daß man eine Synthese in fester Phase ausführt, indem man auf einem Träger vom Typ vernetztes Polystyrol nacheinander die ordnungsgemäß geschützten Aminosäuren mit Hilfe eines Kupplungsmittels einführt, die Schutzgruppen der Aminosäuren entfernt und die so gebildete Peptidkette aus dem Harz freisetzt, dann gegebenenfalls die Disulfidbrücke oder -brücken cyclisiert, um das so angestrebte Peptidderivat zu erhalten.

4. Verfahren gemäß Anspruch 3, dadurch gekennzeichnet, daß:
- der Träger vom Typ vernetztes Polystyrol ein Harz vom Typ "Boc-Trp-CM" ist, in dem Boc eine tert-Butyloxycarbonylgruppe ist, Trp eine Tryptophylgruppe ist und CM den Träger aus vernetztem Polystyrol bezeichnet,
- das Kupplungsmittel (Benzotriazol-1-yloxy)-tris-(dimethylamino)-phosphoniumhexafluorophosphat oder Bop ist,
- die Freisetzung der Peptidkette vom Träger sowie die Entfernung der Schutzgruppen der Aminosäuren mittels Fluorwasserstoffsäure ausgeführt wird, wobei man bei niedriger Temperatur, vorzugsweise bei etwa 0 °C arbeitet.

5. Medikamente, dadurch gekennzeichnet, daß sie aus den Peptidderivaten wie durch die allgemeine Formel (I) des Anspruchs 1 definiert bestehen.

6. Medikamente, dadurch gekennzeichnet, daß sie aus den Peptidderivaten wie in Anspruch 2 definiert bestehen.

7. Pharmazeutische Zusammensetzungen, dadurch gekennzeichnet, daß sie als Wirkstoff wenigstens eines der Medikamente wie in einem der Ansprüche 5 und 6 definiert enthalten.

8. Verwendung des [Nle-7]-ET1-Derivats für die Herstellung eines Medikaments zur Hemmung des Hochdrucks, erzeugt von Endothelin ohne gefäßverengende Wirkung des Endothelins.

## Patentansprüche (Patentansprüche für folgende(n) Vertragsstaat(en): ES, GR)

1. Verfahren zur Herstellung von Peptidderivaten, die der allgemeinen Formel (I):
R1 - H X1 D X2 I X3 (I)
entsprechen, in der X1 einen Leucin-, Arginin- oder Glutaminrest darstellt, X2 einen Isoleucin- oder Valinrest darstellt, X3 einen Tryptophan-, Amidotryptophan- oder D-Naphthylalaninrest darstellt und R1 einen Rest aus 15 Aminosäuren der Formel (la): C S C S S L X4 D K E C X5 Y F C - darstellt, in dem X4 in Position 7 einen Glycin-, Norleucin-, Valin-, Isoleucin-, Leucin-, Alanin-, Phenylalanin-, beta-Alanin- oder Methioninrest darstellt, X5 in Position 12 einen Valin-, Leucin- oder Glutaminsäurerest darstellt und die punktierte Linie gegebenenfalls eine Disulfidbrücke zwischen 2 Cysteinresten darstellt, wobei es sich versteht, daß X4 keinen Methioninrest darstellen kann, wenn X5 einen Valinrest darstellt;
mit Ausnahme des Derivats [Nle-7]-ET1, dadurch gekennzeichnet, daß man eine Synthese in fester Phase ausführt, indem man auf einem Träger vom Typ vernetztes Polystyrol nacheinander die ordnungsgemäß geschützten Aminosäuren mit Hilfe eines Kupplungsmittels einführt, die Schutzgruppen der Aminosäuren entfernt und die so gebildete Peptidkette aus dem Harz freisetzt, dann gegebenenfalls die Disulfidbrücke oder -brücken cyclisiert, um das so angestrebte Peptidderivat zu erhalten.

2. Verfahren gemäß Anspruch 1, dadurch gekennzeichnet, daß:
- der Träger vom Typ vernetztes Polystyrol ein Harz vom Typ "Boc-Trp-CM" ist, in dem Boc eine tert-Butyloxycarbonylgruppe ist, Trp eine Tryptophylgruppe ist und CM den Träger aus vernetztem Polystyrol bezeichnet,
- das Kupplungsmittel (Benzotriazol-1-yloxy)-tris-(dimethylamino)-phosphoniumhexafluorophosphat oder Bop ist,
- die Freisetzung der Peptidkette vom Träger sowie die Entfernung der Schutzgruppen der Aminosäuren mittels Fluorwasserstoffsäure ausgeführt wird, wobei man bei niedriger Temperatur, vorzugsweise bei etwa 0 °C arbeitet.

3. Verfahren gemäß Anspruch 1, dadurch gekennzeichnet, daß die Peptidderivate aus einem der folgenden Peptidderivate bestehen: 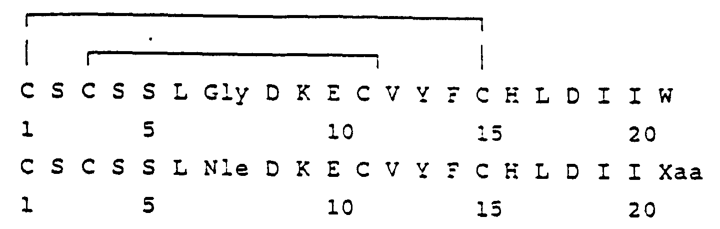
C S C S S L Gly D K E C V Y F C H L D I I W
1       5               10          15          20
C S C S S L Nle D K E C V Y F C H L D I I Xaa
1       5               10          15          20
 worin Xaa D-Naphthylalanin darstellt
